**Europäisches Patentamt**

**European Patent Office**

**Office européen des brevets**

(19)

(11) Veröffentlichungsnummer: **0 205 132 B1**

(12) # EUROPÄISCHE PATENTSCHRIFT

(45) Veröffentlichungstag der Patentschrift: **09.10.91**

(51) Int. Cl.5: **A61F 2/34**

(21) Anmeldenummer: **86107787.3**

(22) Anmeldetag: **07.06.86**

(54) **Pfanne für ein künstliches Hüftgelenk.**

(30) Priorität: **08.06.85 DE 3520663**

(43) Veröffentlichungstag der Anmeldung:
**17.12.86 Patentblatt 86/51**

(45) Bekanntmachung des Hinweises auf die
Patenterteilung:
**09.10.91 Patentblatt 91/41**

(84) Benannte Vertragsstaaten:
**FR GB IT SE**

(56) Entgegenhaltungen:
**EP-A- 0 083 708**
**DE-B- 2 365 022**
**FR-A- 2 416 004**

(73) Patentinhaber: **GMT Gesellschaft für medizini-**
**sche Technik mbH**
**Holstenstrasse 2**
**W-2000 Hamburg 50(DE)**

(72) Erfinder: **Engelbrecht, Eckart, Dr. med.**
**Andreasstrasse 33**
**W-2000 Hamburg 60(DE)**
Erfinder: **Hahn, Michael**
**Niendorfer Kirchenweg 7**
**W-2000 Hamburg 61(DE)**

(74) Vertreter: **Heldt, Gert, Dr. Dipl.-Ing.**
**Neuer Wall 59 III**
**W-2000 Hamburg 36(DE)**

## Beschreibung

Die Erfindung betrifft eine Pfanne für ein künstliches Hüftgelenk mit einer Schale, die in einer Höhlung eines sie aufnehmenden Beckenknochens zementlos verankerbar ist, beim Einsetzen in die Höhlung deren geringem Einlaßquerschnitt anpaßbar und innerhalb der Höhlung auf ein von deren innerer Oberfläche vorgegebenes Maß erweiterbar ist.

Eine derartige Pfanne (europäische Patentanmeldung 83 708) besitzt den Nachteil, daß ihre Schale nicht unmittelbar in der Höhlung des Beckenknochens befestigt werden kann. Vielmehr muß zunächst in die Höhlung eine Hülse eingebracht werden, die einen konischen Innenhohlraum aufweist und mit einer konisch gestalteten Außenfläche in der Höhlung anliegt. Diese Hülse ist in ihrer Längsrichtung geschlitzt, so daß sie mit Hilfe einer ebenfalls konisch ausgebildeten Oberfläche einer Schale so ausgeweitet werden kann, daß sie fest auf die innere Oberfläche der Höhlung gedrückt wird. Je nach der Größe dieser Höhlung dringt die Schale mehr oder minder tief in den Innenhohlraum der Hülse ein. Innerhalb der Höhlung des Beckenknochens soll die eingebrachte Pfanne durch ein- oder anwachsendes Gewebe gehalten werden.

Bis dieses Anwachsen der Pfanne erfolgt ist, sitzt diese nicht fest in der Höhlung. Sie kann sich unter den über das Hüftgelenk zu übertragenden Kräften leicht Verschieben. Darüber hinaus muß eine sehr große Höhlung hergestellt werden, damit die Hülse in dieser befestigt werden kann. Die angestrebte sparsame Resektion von Knochengewebe ist daher nicht möglich.

Aufgabe der vorliegenden Erfindung ist es daher, die Pfanne der einleitend genannten Art so zu verbessern, daß sie einerseits fest in der Höhlung eines Beckenknochens verankert werden kann und andererseits ohne Schädigung des Knochens relativ schnell aus der Höhlung wieder ausgebaut werden kann.

Diese Aufgabe wird erfindungsgemäß dadurch gelöst, daß die Schale aus mindestens zwei Schalenteilen besteht, die gegeneinander beweglich gelagert sind.

Durch diese Ausbildung der Schale ist es möglich, deren Querschnitt in einem weiten Rahmen demjenigen der im Beckenknochen vorgesehenen Höhlung anzupassen. Durch die Verschieblichkeit der Schalenteile wird erreicht, daß die Schale unmittelbar fest in der Höhlung verankert werden kann, ohne daß ein Anwachsen der Schale abgewartet werden muß. Vielmehr kann durch die Verschieblichkeit der Schalenteile erreicht werden, daß diese bei Bedarf auch leicht aus der Höhlung wieder herausgenommen werden kann, wenn beispielsweise im Rahmen einer Reoperation ein neues Hüftgelenk eingesetzt werden muß. Die Ausbildung der Schale mit Hilfe von gegeneinander beweglich gelagerten Schalenteilen besitzt mithin die wesentlichen Vorteile, daß die Schale schnell und sicher in die Höhlung eingesetzt und bei Bedarf dieser auch so wieder entnommen werden kann.

Gemäß einer bevorzugten Ausführungsform der Erfindung sind die Schalenteile schwenkbar gegeneinander gelagert. Durch diese schwenkbare Lagerung werden die Schalenteile leicht in die gewünschte Position gebracht, ohne daß sehr viel Knochenmasse aus dem Beckenknochen entfernt werden muß, um die Höhlung herzustellen. Gemäß einer weiteren bevorzugten Ausführungsform der Erfindung sind die Schalenteile an einem Punkt über ein Schwenkgelenk verschwenkbar gegeneinander gelagert und an einem anderen Punkt ist eine die Schalenteile voneinander abdrückende Abdrückvorrichtung vorgesehen. Auf diese Weise kann einfach und sicher die zur Befestigung der Schale notwendige Kraft in die Schale eingeleitet werden.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

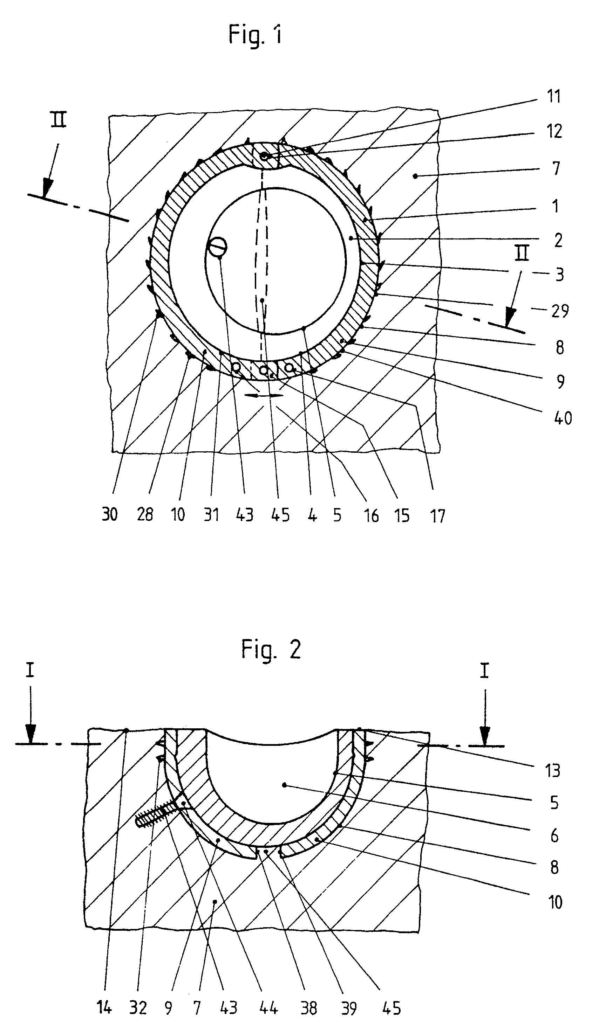

Fig. 1 :    Einen Längsschnitt durch einen Hüftknochen im Bereich einer Hüftgelenkpfanne entlang der Schnittlinie I-I in Figur 2,

Fig. 2 :    einen Querschnitt durch einen Teil eines Beckenknochens entlang der Schnittlinie II-II in Figur 1,

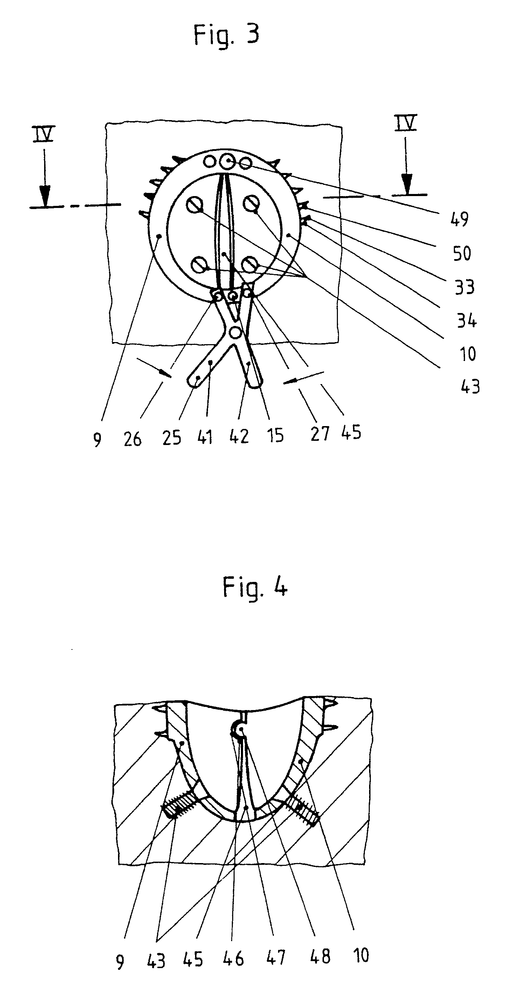

Fig. 3 :    eine Draufsicht auf einen Beckenknochen,

Fig. 4 :    einen Querschnitt durch einen Beckenknochen entlang der Schnittlinie IV-IV in Figur 3,

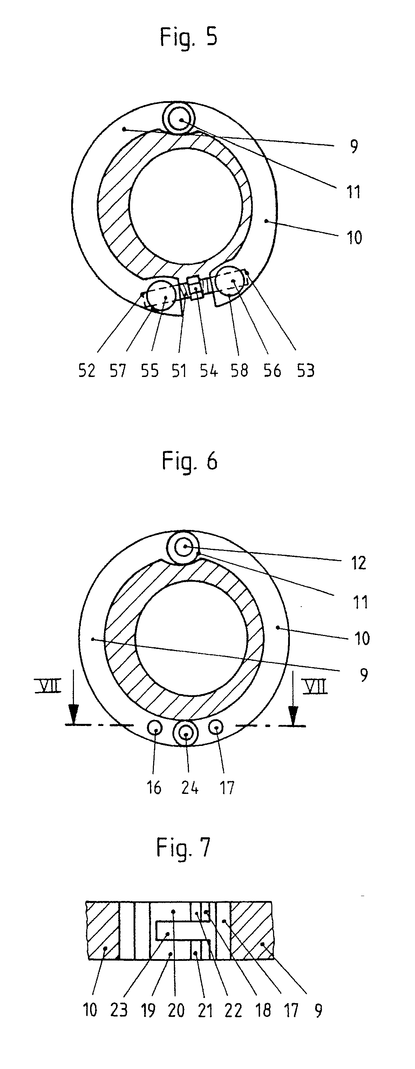

Fig. 5 :    eine Draufsicht auf eine Pfanne,

Fig. 6 :    eine Draufsicht auf eine andere Pfanne,

Fig. 7 :    einen Teilschnitt entlang der Schnittlinie VII-VII in Figur 6,

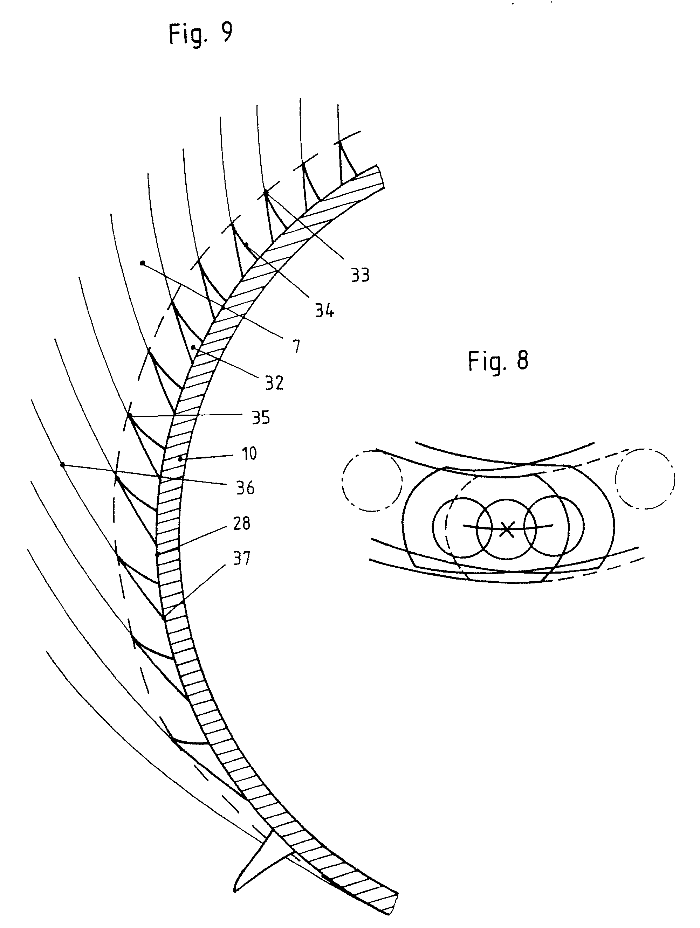

Fig. 8 :    eine Draufsicht auf eine Vergrößerung der Darstellung in Figur 7 und

Fig. 9 :    eine Ausschnittsvergrößerung von einem Querschnitt durch eine Schale.

Eine Pfanne besteht im wesentlichen aus einer Schale 1 und einer elastischen Einlage 2. Die elastische Einlage 2 liegt mir ihrer der Schale 1 zugewandten äußeren Oberfläche 3 unmittelbar an einer ihr zugewandten Innenfläche 4 der Schale 1. Dabei ist die äußere Oberfläche 3 genau auf die Innenfläche 4 angepaßt , so daß sich die Oberfläche 3 der Einlage 2 großflächig auf der Innenfläche 4 der Schale 1 abstützt. Zu diesem Zwecke sind sowohl die Oberfläche 3 als auch die Innenfläche 4 in Form von Halbkugeln ausgebildet. Auf ihrer der Oberfläche 4 abgewandten Innenseite 5 ist die Einlage 2 als Auflager für einen nicht dargestellten Kugelkopf ausgebildet, der in einen von der elastischen Einlage 2 ausgebildeten Innenraum 6 hineinragt. In diesem Innenraum 6 kann der mit einem nicht dargestellten menschlichen Oberschenkel verbundene Kugelkopf je nach der Bewegung des Oberschenkels Schwenkbewegungen ausführen.

Die dabei von der Einlage 2 zu übertragenden Kräfte werden von der Einlage 2 großflächig auf die Schale 1 übertragen, die sich ihrerseits in einem Beckenknochen 7 abstützt. In diesem Beckenknochen 7 ist eine Höhlung 8 ausgebildet, in die die Schale 1 eingepaßt ist.

Zum Zwecke der Einpassung ist die Schale 1 in Form von zwei Schalenhälften 9, 1o ausgebildet, die über ein Schwenkgelenk 11 miteinander verbunden sind. Dieses Schwenkgelenk 11 kann als ein mit einem Bolzen 12 ausgerüstetes Scharnier ausgebildet sein. Um den Bolzen 12 ist die eine Schalenhälfte 9 gegenüber der anderen Schalenhälfte 1o verschwenkbar gelagert.

Das Schwenkgelenk 11 ist in einer oberen Ebene 13 der Schale 1 ausgebildet. Diese obere Ebene 13 schließt mit einer Oberfläche 14 des Beckenknochens 7 bündig ab, in die die Höhlung 8 eingebracht ist. Gegenüber dem Schwenkgelenk 11 ist in der oberen Ebene 13 eine Abdrückvorrichtung 15 vorgesehen, mit deren Hilfe die Schalenhälften 9, 1o um das Schwenkgelenk 11 verschwenkt werden können. Diese Abdrückvorrichtung 15 besteht im wesentlichen aus zwei Bohrungen 16, 17, die quer zur Bewegungsrichtung der um das Schwenkgelenk 11 verschwenkten Schalenhälften 9, 1o in je eine von diesen abgesenkt sind. Zwischen diesen beiden Bohrungen 16, 17 ragt die eine Schalenhälfte 9 mit einer Lasche 18 zwischen zwei Vorsprünge 19, 2o, die gabelförmig von der anderen Schalenhälfte 1o in Richtung auf die Schalenhälfte 9 hervorragen. Zwischen den beiden Vorsprüngen 19, 2o ist ein Abstand vorgesehen, dessen Breite der Dicke der Lasche 18 entspricht.

Durch die Vorsprünge 19, 2o erstrecken sich Bohrungen 21, 22, die quer zur Bewegungsebene der Schalenhälften 9, 1o miteinander fluchten. Darüber hinaus erstreckt sich in gleicher Richtung eine Bohrung 23, durch die Lasche 18, die jedoch nur im auseinandergedrückten Zustand der Schale 1 mit den Bohrungen 21, 22 fluchtet, so daß in diesem Zustand ein Bolzen 24 sich durch sämtliche drei Bohrungen 21, 22; 23 erstrecken kann. Dieser Bolzen 24 hält die Schalenhälften 9, 1o in einem voneinander abgedrückten Zustand fest, so daß sie sich großflächig in die Höhlung 8 einfügen. Um den Bolzen 24 in die Bohrungen 21, 22; 23 einführen zu können, müssen die beiden Schalenhälften 9, 1o voneinander abgedrückt werden. Zu diesem Zwecke wird eine Abdrückzange 25 mit zwei entsprechend ausgebildeten Vorsprüngen 26, 27 in die Bohrungen 16, 17 eingeführt. Sodann können mit Hilfe der Abdrückzange 25 die beiden Schalenhälften 9, 1o um das Schwenkgelenk 11 solange verschwenkt werden, bis der Bolzen 24 in die Bohrungen 21, 22; 23 eingeführt werden kann. In diesem verspannten Zustand der Schalenhälften 9, 1o kann die Abdruckzange 25 aus den Bohrungen 16, 17 abgezogen werden. Beim Verspannen der Schalenhälften 9, 1o wird im Bereich von deren der Höhlung 8 zugewandten Außenflächen 28, 29 nicht nur ein Kraftschluß in der Höhlung 8 herbeigeführt. Vielmehr sind auf den Außenflächen 28, 29 Vorsprünge 3o vorgesehen, die sich beim Verspannen der Schalenhälften 9, 1o in eine ihnen zugewandte Innenfläche 31 der Höhlung 8 hineindrücken. Diese Vorsprünge 3o können als Zähne 32 ausgebildet sein, die mit den Außenflächen 28, 29 fest verbunden sind. Diese Zähne sind mit Schneidkanten 33, 34 versehen, die sich in den Beckenknochen 7 hineinschneiden. Die Schneidkanten 33, 34 sind so gegeneinander ausgerichtet, daß sie an ihrem den Außenflächen 28, 29 abgewandten Ende mit einer scharfen Spitze 35 versehen sind. Diese scharfe Spitze 35 bohrt sich beim Verspannen der Schalenhälften 9, 1o in den Beckenknochen 7 hinein.

Die Zähne 32 sind in der Weise ausgebildet, daß eine ihrer Schneidkanten 34 in Richtung der von den Schalenteilen 9, 1o beim Erweitern der Schale 1 durchgeführten Bewegung verlaufen. Zu diesem Zwecke sind die Schneidkanten 34 der Zähne 32 als Ausschnitte von Kreisbögen 36 ausgebildet. Dieser Kreisbogen 36 hat einen Radius, der gleich der Entfernung ist, die sich zwischen dem Bolzen 12 des Schwenkgelenkes 11 und einem Fußpunkt 37 erstreckt, an dem die Schneidkante 34 in die Außenfläche 28 der Schale 1 einmündet.

Demgegenüber ist die dem Bolzen 12 zugewandte zweite Schneidkante 33 eines jeden Zahnes 32 als eine Abstützung der scharfen Spitze 35 ausgebildet. Diese Schneidkante 33 unterstützt die scharfe Spitze 35, damit diese sich unter dem Einfluß des beim Verspannen der Schalenhälften 9, 1o entstehenden Druckes sich beim Eindringen in den Beckenknochen 7 nicht verformt. Die Ausbildung der ersten Schneidkante 34 in Form eines Kreisbogens 36 hat den Vorteil, daß die Zähne 32 beim Eindringen in den Beckenknochen 7 eine relativ kleine Ausnehmung im Beckenknochen 7 ausbilden, in der der diese Ausnehmung ausbildende Zahn 32 sicher geführt ist.

Durch diese Ausbildung der Zähne 32 besitzen die ersten Schneidkanten 34 im Bereich der Abdrückvorrichtung 15 eine geringere Steigung als die entsprechenden Schneidkanten 34 der im Bereich des Schwenkgelenkes 11 vorgesehenen Zähne. Im Bereich des Schwenkgelenkes 11 können die Zähne 32 relativ steil stehen, da in diesem Bereich die Schalenhälften 9, 1o beim Verschwenken relativ kleine Wege zurücklegen. Zweckmäßigerweise sind im unmittelbaren Bereich der Abdrückvorrichtung 15 überhaupt keine Zähne 32 vorgesehen. Darüber hinaus können im Bereich des Schwenkgelenkes 11 die Zähne 32 relativ eng beieinander angeordnet sein. Die Zähne 32 können in Reihen angeordnet sein, die in Form von Breitengraden oder in Form von Längengraden über die Außenfläche 28, 29 verteilt sein. Es ist jedoch auch möglich, daß die Reihen schräg sowohl zu den Breitengraden als auch zu den Längengraden verlaufen. Im Regelfall reicht für die Sicherung der Schale 1 eine Anordnung der Zähne 32 aus, bei der sich die Zähne 32 lediglich in zwei parallel zueinander verlaufenden Reihen unmittelbar unterhalb der oberen Ebene 13 der Schale 1 in Form von Breitengraden erstrecken.

Die Schale 1 besteht zweckmäßigerweise aus einem hochfesten Material, beispielsweise Stahl. Sie kann im Bereich der höchsten Belastungen mit einer dikkeren Wandstärke steifer ausgebildet sein als im Bereich niedriger Beanspruchungen. Demgegenüber besteht die elastische Einlage 2 aus einem Kunststoff, beispielsweise einem Polyäthylen.

Vor dem Einbau der Schale 1 wird die Höhlung 8 im Beckenknochen 7 sorgfältig vorbereitet und mit einer ebenen Oberfläche versehen. In diese Höhlung 8 wird die Schale 1 eingesetzt, wobei die Schalenhälften 9, 1o zunächst unverspannt sind und mit ihren Berührungslinien 38, 39 unmittelbar aufeinander stossen. In diesem Zustand kann die Schale 1 durch einen im Beckenknochen 7 ausgebildeten Einlaßquerschnitt 4o in die Höhlung 8 eingeführt werden.

Innerhalb der Höhlung 8 wird die Schale 1 genau ausgerichtet, so daß sie den nicht dargestellten Gelenkkopf eines Oberschenkelknochens aufnehmen kann, Nachdem diese Ausrichtung erfolgt ist, wird die Abdrückzange 25 mit ihren Vorsprüngen 26, 27 in die Bohrungen 16, 17 eingeführt. Sodann werden die Schalenhälften 9, 1o in der Höhlung 8 mit Hilfe der Abdrückzange 25 verspannt, in dem ihre Zangenschenkel 41, 42 in Richtung aufeinander zu gedrückt werden, so daß die Vorsprünge 26, 27 sich voneinander entfernen. Dadurch führen die Schalenhälften 9, 1o Schwenkbewegungen um das Schwenkgelenk 11 aus, bis die Bohrungen 21, 22; 23 miteinander fluchten. Sodann wird durch diese Bohrungen 21, 22; 23 der Bolzen 24 eingeführt. Während des Verspannens haben die Zähne 32 in den Beckenknochen 7 eingeschnitten. Auf diese Weise ist die Schale 1 form- und kraftschlüssig fest in der Höhlung 8 festgelegt.

In ähnlicher Weise kann eine Festlegung der Schalenhälften 9, 1o auch dadurch herbeigeführt werden, daß diese mit Knochenschrauben 43 im Beckenknochen 7 festgelegt werden. Diese Knochenschrauben 43 erstrecken sich durch Bohrungen 44, die in den Schalenhälften 9, 1o zu diesem Zwecke vorgesehen sind. Dabei werden die Knochenschrauben 43 in solche Teile des Beckenknochens 7 eingedreht, in denen dieser durch eine entsprechend große Ansammlung von hochfester Knochenmasse am Widerstandsfähigsten ist. Die Verwendung von Knochenschrauben 43 ist möglich zusätzlich zu der Verspannung der Schalenhälften 9, 1o mit Hilfe der Abdrückvorrichtung 15. Es ist auch möglich, eine Sicherung der Schalenhälften 9, 1o ausschließlich mit Knochenschrauben 43 vorzunehmen.

Nach der Befestigung der Schalenhälften 9, 1o wird in die Schale 1 die elastische Einlage 2 eingelegt. Diese überdeckt nicht nur die Knochenschraube 43, sondern auch einen sich zwischen den Schalenhälften 9, 1o ausbildenden Spalt 45, der dadurch entsteht, daß die beiden Schalenhälften 9, 1o voneinander abgedrückt werden. Dieser Spalt 45 verläuft in einem Bereich der Schale 1, der am wenigsten bei der Übertragung von Kräften aus dem Beckenknochen 7 in den nicht dargestellten Oberschenkel belastet ist. Dieser Spalt 45 kann auch im Bereich der oberen Ebene 13 eine besondere Führung 46 aufweisen. Diese kann als eine Nut 47 in einer der beiden Schalenhälften 9 ausgebildet sein, in die eine Feder 48 der anderen Schalenhälfte 1o hineinragt.

Schließlich ist es auch denkbar, die beiden Schalenhälften 9, 1o nicht über ein Schwenkgelenk 11 miteinander zu verbinden, sondern vielmehr parallel zueinander zu verschieben. Diese Parallelverschiebung kann entweder mit mindestens zwei Abdrückvorrichtung 15, 49 vorgenommen werden, die an zwei einander möglichst gegenüberliegenden Punkten der oberen Ebene 13 anzubringen sind. Es ist jedoch auch möglich, statt zwei Abdrückvorrichtungen 15, 49 nur eine Abdrückvorrichtung vorzusehen und die Schalenhälften 9, 1o auf Führungen anzuordnen, auf denen sie mit Hilfe einer Abdrückvorrichtung 15 voneinander entfernt werden können. Auf diese Weise entsteht zwischen den beiden Schalenhälften 9, 1o ein etwa gleichbleibend breiter Spalt 45. Die Verspannung der Schalenhälften 9, 1o erfolgt in diesen Fällen mit zwei gleichzeitig zur Anwendung kommenden Abdrückzangen 25. Bei derartig parallel geführten Schalenhälften 9, 1o werden die Vorsprünge 19, 2o in Form von Kegeln 5o ausgebildet, deren Mittelsenkrechte in Richtung der Bewegungen verläuft, die die Schalenhälften 9, 1o beim Verspannen zurücklegen. Eine Krümmung der Schneidkanten 33, 34 ist nicht notwendig, da diese in Richtung der Mittelsenkrechten in den Beckenknochen 7 eindringen, so daß sie von allen Seiten gleichmäßig von Knochenmasse umgeben sind.

Die Abdrückvorrichtung 15 kann auch mit Hilfe eines Schraubbolzens 51 ausgebildet sein, der an einem seiner beiden Enden 52 mit Rechts- und am anderen Ende 53 mit Linksgewinde ausgebildet ist. Zwischen den beiden Enden 52, 53 befindet sich ein als Kupplung für ein Werkzeug ausgebildeter Sechskant 54.

Die beiden Enden 53, 54 ragen jeweils in Muttern 55, 56 hinein, von denen jede dem jeweiligen Gewinde des Schraubbolzens 51 zugeordnet ist. Die Muttern 55, 56 sind quer zur Richtung des Schraubbolzens 54 in entsprechenden Bohrungen 57, 58 der Schalenhälften 9, 1o schwenkbar gelagert. Auf diese Weise können sie sich den Schwenkbewegungen der Schalenhälften 9, 1o anpassen. Durch Drehungen des Schraubbolzens 51 schrauben sich die jeweiligen Muttern 55, 56 entweder in Richtung auf den Sechskant 54 weiter auf den Schraubbolzen 51 auf oder in entgegengesetzte Richtung von diesem ab. Beim Abschrauben werden die Schalenhälften 9, 1o in die Höhlung 8 hineingepreßt und damit verspannt. In entgegengesetzter Richtung wird der Schraubbolzen 51 gedreht, um die Schalenhälften 9, 1o aus ihrer Verspannung zu lösen und sie aus der Höhlung 8 herauszunehmen. Es ist auch möglich, durch Verwendung von zwei Schraubbolzen 51 an zwei einander gegenüberliegenden Seiten der Schale Parallelverschiebungen der Schalenhälften 9, 1o durchzuführen. Zusätzlich zu dieser mit dem Schraubbolzen 51 ausgerüsteten Abdrückvorrichtung 15 kann auch eine Befestigung der Schalenhälften 9, 1o mit Hilfe von Knochenschrauben 43 erfolgen.

**Patentansprüche**

1. Pfanne für ein künstliches Hüftgelenk mit einer Schale (1), die in einer Höhlung (8) eines sie aufnehmenden Beckenknochens zementlos verankerbar ist, beim Einsetzen in die Höhlung (8) deren geringerem Einlaßquerschnitt (40) anpaßbar und innerhalb der Höhlung (8) auf ein von deren innerer Oberfläche (31) vorgegebenes Maß erweiterbar ist, dadurch gekennzeichnet, daß die Schale (1) aus mindestens zwei Schalenteilen (9,10) besteht, die gegeneinander beweglich gelagert sind.

2. Pfanne nach Anspruch 1 , dadurch gekennzeichnet, daß die Schalenteile (9, 1o) schwenkbar gegeneinander gelagert sind.

3. Pfanne nach Anspruch 1 und 2, dadurch gekennzeichnet, daß die Schalenteile (9, 1o) an einem Punkt über ein Schwenkgelenk (11) verschwenkbar gegeneinander gelagert sind und an einem anderen Punkt eine die Schalenteile (9, 1o) voneinander abdrückende Abdrückvorrichtung (15) vorgesehen ist:

4. Pfanne nach Anspruch 3 , dadurch gekennzeichnet, daß die Abdrückvorrichtung (15) und das Schwenkgelenk (11) in einer durch die Schale (1) verlaufenden Ebene angeordnet sind.

5. Pfanne nach Anspruch 4 , dadurch gekennzeichnet, daß die Ebene (13) durch eine die Schale (1) öffnende Einsatzöffnung verläuft.

6. Pfanne nach Anspruch 3 bis 5, dadurch gekennzeichnet, daß das Schwenkgelenk (11) als ein Bolzen (12) ausgebildet ist, um den die Schalenteile (9, 1o) verschwenkbar gelagert sind.

7. Pfanne nach Anspruch 1 , dadurch gekennzeichnet, daß die Schalenteile (9, 1o) verschieblich gegeneinander gelagert sind.

8. Pfanne nach Anspruch 7, dadurch gekennzeichnet, daß die Schalenteile (9, 1o) auf parallel zueinander verlaufenden Bahnen verschieblich angeordnet sind.

9. Pfanne nach Anspruch 7 und 8, dadurch gekennzeichnet, daß zwischen den Schalenteilen (9, 1o) mindestens eine Abdrückvorrichtung (15) vorgesehen ist und die Schalenteile (9,1o) auf Führungen gelagert sind, die parallel zueinander verlaufen.

10. Pfanne nach Anspruch 7 und 8, dadurch gekennzeichnet, daß zwischen den Schalenteilen (9, 1o) zwei parallel zueinander angeordnete Abdrückvorrichtungen (15) vorgesehen sind.

11. Pfanne nach Anspruch 10 , dadurch gekennzeichnet, daß die Schalenteile (9, 1o) auf parallel zueinander verlaufenden Führungen gelagert sind.

12. Pfanne nach Anspruch 3 bis 11, dadurch gekennzeichnet, daß die Abdrückvorrichtung (15) als Schraubbolzen ausgebildet ist, der an seinem einen Ende (52) ein Links- und an seinem anderen Ende (53) ein Rechtsgewinde aufweist und jedes Ende (52, 53) in einer dem jeweiligen Gewinde angepaßten Mutter (55, 56) drehbar gelagert ist, von denen jede in einer der beiden Schalenhälften (9, 1o) befestigt ist.

13. Pfanne nach Anspruch 12 , dadurch gekennzeichnet, daß der Schraubbolzen (51) in seiner zwischen seinen beiden Enden (52, 53) liegenden Mitte mit einer Kupplung zum Ankuppeln von Werkzeug versehen ist.

14. Pfanne nach Anspruch 12 , dadurch gekennzeichnet, daß jede der Muttern (55, 56) verschwenkbar in je einer der Schalenhälften (9, 1o) gelagert ist.

15. Pfanne nach Anspruch 3 bis 12, dadurch gekennzeichnet, daß als Abdrückvorrichtung (15) eine Kupplung vorgesehen ist, die in jedem ihrer Kupplungsteile mindestens eine quer sich zur Bewegungsrichtung der sich voneinander abdrückenden Schalenteile (9, 1o) erstreckende Bohrung (21, 22; 23) aufweist und daß sich durch im voneinander abgedrückten Zustand der Schalenteile (9, 1o) fluchtenden Bohrungen (21, 22; 23) ein Bolzen (24) erstreckt.

16. Pfanne nach Anspruch 15 , dadurch gekennzeichnet, daß an den Kupplungsteilen mindestens eine ein Abdrückwerkzeug (25) aufnehmende Angriffsfläche ausgebildet ist.

17. Pfanne nach Anspruch 16 , dadurch gekennzeichnet, daß als Angriffsfläche in jedem Kupplungsteil mindestens eine sich quer zur Bewegungsrichtung der Kupplungsteile erstreckende Bohrung (16, 17) vorgesehen ist, in die Abdrückwerkzeug (25) mit entsprechenden Vorsprüngen (26, 27) hineinfaßt.

18. Pfanne nach Anspruch 1 bis 17, dadurch gekennzeichnet, daß die Schalenteile (9, 1o) auf ihren dem Beckenknochen (7) zugewandten Außenflächen (28, 29) mit Vorsprüngen (30) für ihre formschlüssige Verbindung mit dem Beckenknochen (7) versehen sind.

19. Pfanne nach Anspruch 18 , dadurch gekennzeichnet, daß die Vorsprünge (30) aus Zähnen (32) bestehen, die beim Erweitern der Schale (1) in den Beckenknochen (7) hineinragen.

20. Pfanne nach Anspruch 19 , dadurch gekennzeichnet, daß die Zähne (32) fest mit den Außenflächen (28, 29) verbunden sind.

21. Pfanne nach Anspruch 19 u. 20, dadurch gekennzeichnet, daß die Zähne (32) in den Beckenknochen (7) hineinschneidende Schneidkanten (33, 34) aufweisen, die in einer dem Beckenknochen (7) zugewandten scharfen Spitze (35) zulaufen.

22. Pfanne nach Anspruch 21 dadurch gekennzeichnet, daß mindestens eine der einen Zahn (32) bildenden Schneidkanten (33, 34) in Richtung der von den Schalenteilen (9, 1o) beim Erweitern der Schale (1) durchgeführten Bewegungen verlaufen.

23. Pfanne nach Anspruch 4, 21 u. 22, dadurch gekennzeichnet, daß bei um das Schwenkgelenk (11) verschwenkbaren Schalenteilen (9, 1o) die jeweils dem Schwenkgelenk (11) abgewandte Schneidkante (34) eines jeden Zahnes (32) in Richtung eines Kreisbogens (36) verläuft, dessen Radius gleich der Entfernung ist, die sich zwischen dem Bolzen (12) des Schwenkgelenkes (11) und einem Fußpunkt (37) erstreckt, an dem die Schneidkante (34) in die Außenfläche (28, 29) einmündet.

24. Pfanne nach Anspruch 4, 21 bis 23 dadurch gekennzeichnet, daß die dem Schwenkgelenk (11) jeweils zugewandte Schneidkante (33) eines jeden Zahnes (32) eine die Spitze (35) des Zahnes (32) ausreichend abstützende Steigung aufweist.

25. Pfanne nach Anspruch 4, 19 bis 24 dadurch gekennzeichnet, daß im Bereich des Schwenkgelenkes (11) eine engere Zahnfolge vorgesehen ist als im Bereich der Abdrückvorrichtung (15).

26. Pfanne nach Anspruch 11 bis 25, dadurch gekennzeichnet, daß im Bereich der Abdrückvorrichtung (15) die Außenfläche (28, 29) zahnlos ausgebildet ist.

27. Pfanne nach Anspruch 9 bis 26, dadurch gekennzeichnet, daß bei parallel zueinander verschobenen Schalenteilen (9, 1o) die Zähne (32) Kegel (5o) ausbilden, deren Mittelsenkrechten weitgehend parallel zueinander verlaufen.

28. Pfanne nach Anspruch 27, dadurch gekennzeichnet, daß die Zähne (32) in Reihen angeordnet sind, die in Form von Breitengraden über die Außenfläche (28, 29) verlaufen.

29. Pfanne nach Anspruch 27, dadurch gekennzeichnet, daß die Zähne (32) in Reihen angeordnet sind, die in Form von Längengraden über die Außenfläche (28, 29) verlaufen.

30. Pfanne nach Anspruch 27 dadurch gekennzeichnet, daß die Zähne (32) in Reihen angeordnet sind, die schräg zu Längen- und Breitengraden verlaufen.

31. Pfanne nach Anspruch 2 bis 30 , dadurch gekennzeichnet, daß jedes der Schalenteile (9, 1o) mit mindestens einer Knochenschraube (43) am Beckenknochen (7) befestigt ist.

32. Pfanne nach Anspruch 31 , dadurch gekennzeichnet, daß die Knochenschraube (43) durch eine im jeweiligen Schalenteile (9, 1o) vorgesehene Bohrung (44) in Richtung der zu übertragenden Kräfte in den Beckenknochen (7) hineinragen.

33. Pfanne nach Anspruch 1 bis 32, dadurch gekennzeichnet, daß die Schale (1) aus einem hochfesten Material besteht.

34. Pfanne nach Anspruch 1 bis 33, dadurch gekennzeichnet, daß in der Schale (1) eine elastische Einlage (2) angeordnet ist.

35. Pfanne nach Anspruch 33 , dadurch gekennzeichnet, daß als hochfestes Material Stahl vorgesehen ist.

36. Pfanne nach Anspruch 34 , dadurch gekennzeichnet, daß als elastische Einlage (2) Polyäthylen vorgesehen ist.

37. Pfanne nach Anspruch 1 bis 36 , dadurch gekennzeichnet, daß die Schale (1) im Bereich ihrer höchsten Belastung eine größere Wandstärke aufweist, als in ihren übrigen Bereichen.

**Claims**

1. A socket for an artificial hip joint comprising a shell (1) which can be cementlessly anchored in a cavity (8) in a hip bone for receiving it, which upon being inserted into the cavity (8) can be adapted to the smaller entrance cross-section (40) thereof and which within the cavity (8) can be enlarged to a dimension which is predetermined by the internal surface (31) thereof, characterised in that the shell (1) comprises at least two shell portions (9, 10) which are mounted movably relative to each other.

2. A socket according to claim 1 characterised in that the shell portions (9, 10) are mounted pivotably relative to each other.

3. A socket according to claim 1 and claim 2 characterised in that the shell portions (9,10) are mounted pivotably relative to each other at a point by way of a pivot connection (11) and provided at another point is a pressing means (15) for pressing the shell portions (9, 10) away from each other.

4. A socket according to claim 3 characterised in that the pressing means (15) and the pivot connection (11) are arranged in a plane passing through the shell (1).

5. A socket according to claim 4 characterised in that the plane (13) passes through an insertion opening which opens the shell (1).

6. A socket according to claims 3 to 5 characterised in that the pivot connection (11) is in the form of a pin (12) about which the shell portions (9, 10) are pivotably mounted.

7. A socket according to claim 1 characterised in that the shell portions (9, 10) are mounted slidably relative to each other.

8. A socket according to claim 7 characterised in that the shell portions (9, 10) are arranged slidably on paths which extend parallel to each other.

9. A socket according to claims 7 and 8 characterised in that at least one pressing means (15) is provided between the shell portions (9, 10) and the shell portions (9, 10) are mounted on guides which extend parallel to each other.

10. A socket to claim 7 and claim 8 characterised in that two pressing means (15) which are arranged parallel to each other are provided between the shell portions (9, 10).

11. A socket according to claim 10 characterised in that the shell portions (9, 10) are mounted on guides which extend parallel to each other.

12. A socket according to claims 3 to 11 characterised in that the pressing means (15) is in the form of a screw pin which has a left-hand thread at its one end (52) and a right-hand thread at its other end (53) and each end (52, 53) is rotatably mounted in a nut (55, 56) adapted to the respective thread, each of which nuts is fixed in one of the two shell halves (9, 10).

13. A socket according to claim 12 characterised in that the screw pin (51) is provided in its centre which lies between its two ends (52, 53) with a coupling means for coupling a tool thereto.

14. A socket according to claim 12 characterised in that each of the nuts (55, 56) is mounted pivotably in a respective one of the shell halves (9, 10).

15. A socket according to claims 3 to 12 characterised in that the pressing means (15) is in the form of a coupling means which in each of its coupling portions has at least one bore (21, 22; 23) which extends transversely with respect to the direction of movement of the shell portions (9, 10) which are pressed away from each other, and that a pin (24) extends through the bores (21, 22; 23) which are aligned when the shell portions (9, 10) are in the condition of being pressed away from each other.

16. A socket according to claim 15 characterised in that provided on the coupling portions is at least one engagement surface for receiving a pressing tool (25) for urging the shell portions away from each other.

17. A socket according to claim 16 characterised in that provided as the engagement surface in each coupling portion is at least one bore (16, 17) which extends transversely with respect to the direction of movement of the coupling portions and into which a pressing tool (25) engages with corresponding projections (26, 27).

18. A socket according to claims 1 to 17 characterised in that on their outside surfaces (28, 29) which are towards the hip bone (7) the shell portions (9, 10) are provided with projections (30) for positive connection thereof to the hip bone (7).

19. A socket according to claim 18 characterised in that the projections (30) comprise teeth (32) which project into the hip bone (7) when the shell (1) is expanded.

20. A socket according to claim 19 characterised in that the teeth (32) are fixedly connected to the outside surfaces (28, 29).

21. A socket according to claims 19 and 20 characterised in that the teeth (32) have cutting edges (33, 34) which cut into the hip bone (7) and which converge in a sharp tip (35) which is towards the hip hone (7).

22. A socket according to claim 21 characterised in that at least one of the cutting edges (33, 34) which form a tooth (32) extend in the direction of the movements performed by the shell portions (9, 10) when the shell (1) is expanded.

23. A socket according to claims 4, 21 and 22 characterised in that, when the shell portions (9, 10) are pivotable about the pivot connection (11), the respective cutting edge (34), which is away from the pivot connection (11), of each tooth (32) extends in the direction of a circular arc (36) whose radius is equal to the distance between the pin (12) of the pivot connection (11) and a base point (37) at which the cutting edge (34) meets the outside surface (28, 29).

24. A socket according to claims 4 and 21 to 23 characterised in that the respective cutting edge (33) which is towards the pivot connection (11), of each tooth (32) is of a gradient which sufficiently supports the tip (35) of the tooth (32).

25. A socket according to claims 4 and 19 to 24 characterised in that a closer succession of teeth is provided in the region of the pivot connection (11) than in the region of the pressing means (15).

26. A socket according to claims 11 to 25 characterised in that the outside surface (28, 29) is without teeth in the region of the pressing means (15).

27. A socket according to claims 9 to 26 characterised in that, when the shell portions (9, 10) are displaced parallel to each other, the teeth (32) form cones (50), the perpendicular lines through the centres thereof extending substantially parallel to each other.

28. A socket according to claim 27 characterised in that the teeth (32) are arranged in rows which extend in the form of degrees of latitude over the outside surface (28, 29).

29. A socket according to claim 27 characterised in that the teeth (32) are arranged in rows which extend in the form of degrees of longitude over the outside surface (28, 29).

30. A socket according to claim 27 characterised in that the teeth (32) are arranged in rows which extend inclinedly relative to degrees of longitudinal and latitude.

31. A socket according to claims 2 to 30 characterised in that each of the shell portions (9, 10) is fixed by at least one bone screw (43) to the hip bone (7).

32. A socket according to claim 31 characterised in that the bone screw (43) projects into the hip hone (7) through a bore (44) in the respective shell portion (9, 10) in the direction of the forces to be transmitted.

**33.** A socket according to claims 1 to 32 characterised in that the shell (1) comprises a high-strength material.

**34.** A socket according to claims 1 to 33 characterised in that a resilient insert (2) is arranged in the shell (1).

**35.** A socket according to claim 33 characterised in that steel is used as the high-strength material.

**36.** A socket according to claim 34 characterised in that polyethylene is used as the resilient insert (2).

**37.** A socket according to claims 1 to 36 characterised in that the shell (1) is of greater wall thickness in the region of its highest loading than in its other regions.

**Revendications**

**1.** Cuvette pour une articulation de hanche artificielle avec une coquille (1), apte à être ancrée sans ciment dans un évidement (8) d'un os du bassin la recevant, à être adaptée lors de l'insertion dans l'évidement (8) au diamètre réduit d'entrée (40) de celui-ci et à être déployée à l'intérieur de l'évidement (8) dans une mesure prédéterminée par la surface intérieure (31) de celui-ci, caractérisée en ce que la coquille (1) se compose d'au moins deux parties de coquille (9,10), qui sont supportées de manière mobile l'une par rapport à l'autre.

**2.** Cuvette selon la revendication 1, caractérisée en ce que les parties de coquille (9,10) sont supportées de manière pivotante l'une par rapport à l'autre.

**3.** Cuvette selon la revendication 1 ou 2, caractérisée en ce que les parties de coquille (9,10) sont supportées de manière pivotante l'une par rapport à l'autre en un point par l'intermédiaire d'une articulation de pivotement (11) et en ce qu'en un autre point un mécanisme d'écartement (15) écartant les parties de coquille (9,10) l'une de l'autre est prévu.

**4.** Cuvette selon la revendication 3, caractérisée en ce que le mécanisme d'écartement (15) et l'articulation de pivotement (11) sont disposés dans un plan passant à travers la coquille (1).

**5.** Cuvette selon la revendication 4, caractérisée en ce que le plan (13) passe à travers une ouverture d'insertion ouvrant la coquille (1).

**6.** Cuvette selon l'une des revendications 3 à 5, caractérisée en ce que l'articulation de pivotement (11) est conçue comme un boulon (12), autour duquel les parties de coquille (9,10) sont supportées de manière pivotante.

**7.** Cuvette selon la revendication 1, caractérisée en ce que les parties de coquille (9,10) sont supportées de manière coulissante l'une par rapport à l'autre.

**8.** Cuvette selon la revendication 7, caractérisée en ce que les parties de coquille (9,10) sont disposées sur des trajets parallèles l'un à l'autre de manière coulissante.

**9.** Cuvette selon l'une des revendications 7 et 8, caractérisée en ce qu'au moins un mécanisme d'écartement (15) est prévu entre les parties de coquille (9,10) et en ce que les parties de coquille (9,10) sont supportées sur des guides qui sont parallèles l'un à l'autre.

**10.** Cuvette selon l'une des revendications 7 et 8, caractérisée en ce que deux mécanismes d'écartement (15) parallèles l'un à l'autre sont prévus entre les parties de coquille (9,10).

**11.** Cuvette selon la revendication 10, caractérisée en ce que les parties de coquille (9,10) sont supportées sur des guides parallèles l'un à l'autre.

**12.** Cuvette selon l'une des revendications 3 à 11, caractérisée en ce que le mécanisme d'écartement (15) est conçu comme un bouton fileté, qui présente à l'une de ses extrémités (52) un filet lévogyre et à son autre extrémité (53) un filet dextrogyre et en ce que chaque extrémité (52,53) est supportée de manière rotative dans un écrou (55,56) adapté au filet correspondant, chaque écrou étant fixé dans l'une des deux moitiés de coquille (9,10).

**13.** Cuvette selon la revendication 12, caractérisée en ce que le bouton fileté (51) est pourvu en son milieu situé entre ses deux extrémités (52,53) d'un dispositif de couplage pour le couplage d'un outil.

**14.** Cuvette selon la revendication 12, caractérisée en ce que chacun des écrous (55,56) est supporté de manière basculante dans l'une des moitiés de coquille (9,10).

**15.** Cuvette selon l'une des revendications 3 à 12, caractérisée en ce que comme mécanisme d'écartement (15) il est prévu un dispositif de couplage qui présente dans chacune de ses parties de couplage au moins un trou (21,22;23) s'étendant perpendiculairement au sens du mouvement des parties de coquille (9,10) s'écartant l'une de l'autre et en ce qu'un boulon (24) s'étend à travers les trous (21,22;23) qui coïncident à l'état écarté l'une de l'autre des parties de coquille (9,10).

16. Cuvette selon la revendication 15, caractérisée en ce qu'au moins une surface de prise recevant un outil d'écartement (25) est prévue sur les parties de couplage.

17. Cuvette selon la revendication 16, caractérisée en ce que comme surface de prise dans chaque partie de couplage il est prévu au moins un trou (16,17) s'étendant perpendiculairement au sens du déplacement des parties de couplage, dans lequel un outil d'écartement (25) avec des saillies correspondantes (26,27) se met en prise.

18. Cuvette selon l'une des revendications 1 à 17, caractérisée en ce que les parties de coquille (9,10) sont pourvues sur leurs surfaces extérieures (28,29) dirigées vers l'os du bassin (7) de saillies (30) pour leur liaison par engagement positif avec l'os du bassin (7).

19. Cuvette selon la revendication 18, caractérisée en ce que les saillies (30) se composent de dents (32) qui dépassent dans l'os du bassin (7) lors du déploiement de la coquille (1).

20. Cuvette selon la revendication 19, caractérisée en ce que les dents (32) sont reliées de manière fixe avec les surfaces extérieures (28,29).

21. Cuvette selon la revendication 19 ou 20, caractérisée en ce que les dents (32) présentent des arêtes coupantes (33,34) pénétrant dans l'os du bassin (7), qui convergent en une pointe aiguë (35) dirigée vers l'os du bassin (7).

22. Cuvette selon la revendication 21, caractérisée en ce qu'au moins une des arêtes coupantes (33,34) formant une dent (32) est orientée dans le sens des mouvements effectués par les parties de coquille (9,10) lors du déploiement de la coquille (1).

23. Cuvette selon l'une des revendications 4,21 et 22, caractérisée en ce que pour les parties de coquille (9,10) pivotant autour d'une articulation de pivotement (11), l'arête coupante (34) orientée à l'opposé de l'articulation de pivotement (11) de chaque dent (32) est orientée dans le sens d'un arc de cercle (36), dont le rayon est égal à la distance qui s'étend entre le boulon (12) de l'articulation de pivotement (11) et un point de base (37) où l'arête coupante (34) aboutit à la surface extérieure (28,29).

24. Cuvette selon l'une des revendications 4, 21 à 23, caractérisée en ce que l'arête coupante (33) dirigée vers l'articulation de pivotement (11) de chaque dent (32) présente une inclinaison soutenant suffisamment la pointe (35) de la dent (32).

25. Cuvette selon l'une des revendications 4,19 à 24, caractérisée en ce que dans le secteur de l'articulation de basculement (11) une succession plus étroite de dents que dans le secteur du mécanisme d'écartement (15) est prévue.

26. Cuvette selon l'une des revendications 11 à 25, caractérisée en ce que dans le secteur du mécanisme d'écartement (15) la surface extérieure (28,29) est conçue sans dents.

27. Cuvette selon l'une des revendications 9 à 26, caractérisée en ce que pour des parties de coquille (9,10) coulissant parallèlement l'une à l'autre, les dents (32) forment des cônes (50) dont les médiatrices sont sensiblement parallèles les unes aux autres.

28. Cuvette selon la revendication 27, caractérisée en ce que les dents (32) sont disposées en rangées qui courent sous forme de parallèles sur la surface extérieure (28,29).

29. Cuvette selon la revendication 27, caractérisée en ce que les dents (32) sont disposées en rangées qui courent sous forme de méridiens sur la surface extérieure (28,29).

30. Cuvette selon la revendication 27, caractérisée en ce que les dents (32) sont disposées en rangées qui courent obliquement par rapport aux méridiens et aux parallèles.

31. Cuvette selon l'une des revendications 2 à 30, caractérisée en ce que chacune des parties de coquille (9,10) est fixée par au moins une vis à os (43) à l'os du bassin (7).

32. Cuvette selon la revendication 31, caractérisée en ce que les vis à os (43) dépassent par un trou (44) prévu dans la partie de coquille (9,10) correspondante dans l'os du bassin (7), dans le sens des forces à transmettre.

33. Cuvette selon l'une des revendications 1 à 32, caractérisée en ce que la coquille (1) se compose de matériau à résistance élevée.

34. Cuvette selon l'une des revendications 1 à 33, caractérisée en ce qu'un revêtement élastique (2) est disposé dans la coquille (1).

35. Cuvette selon la revendication 33, caractérisée en ce que le matériau à résistance élevée utilisé est de l'acier.

36. Cuvette selon la revendication 34, caractérisée en ce que du polyéthylène est prévu comme revêtement élastique (2).

37. Cuvette selon l'une des revendications 1 à 36, caractérisée en ce que la coquille (1) présente dans le secteur de sa plus forte sollicitation une plus grande épaisseur de paroi que dans les autres secteurs.

## Fig. 1

11
12
7
1
2
3
29
8
9
40

30  28  10  31  43  45  4  5  16  15  17

## Fig. 2

13
5
6
8
10

14  32  9  7  43  44  38  39  45

# Fig. 3

# Fig. 4

## Fig. 5

52  57  55  51  54  58  56  53

## Fig. 6

12
11
10
9

VII                    VII

16   24   17

## Fig. 7

10  23  19  20  21  22  18  17  9

Fig. 9

Fig. 8

33

34

7

32

35

10

36

28

37